# EUROPEAN PATENT APPLICATION

(11) **EP 1 839 502 A1**
(43) Date of publication of application: **03.10.2007**
(21) Application number: 05816740.4
(22) Date of filing: 07.12.2005
(51) Int. Cl.: A23L 1/30, A23L 1/305, A61K 31/198

(54) **FINE POWDER OF AMINO ACID AND SUSPENSION THEREOF**

(30) Priority: 07.12.2004 JP 2004354391
(71) Applicant: Ajinomoto Co., Inc., Tokyo 104-8315 (JP)
(72) Inventor: KAMINOYAMA, Meguru, Kanagawa, 2450004 (JP); NISHI, Kazuhiko, Kanagawa, 2340051 (JP); KOUDA, Tohru, Ajinomoto Co., Inc., Kanagawa, 2108681 (JP); TSUJIMOTO, Susumu, Ajinomoto Co., Inc., Kanagawa, 2108681 (JP)
(74) Representative: Nicholls, Kathryn Margaret
(86) International application number: PCT/JP2005/022905
(87) International publication number: WO 2006/062238

(57) **Abstract**

The present invention provides a fine powder of a slightly soluble amino acid and a suspension wherein the fine powder is uniformly and stably dispersed. Specifically, the present invention provides a fine powder of a slightly soluble amino acid having an average particle size of 0.1 - 5 µm, which is obtained by wet milling the slightly soluble amino acid, and a suspension wherein the fine powder is dispersed.

## Description

### Technical Field

The present invention relates to a suspension comprising a slightly soluble amino acid such as cystine, aspartic acid, tyrosine, glutamic acid, tryptophan and the like, which is uniformly and stably dispersed therein.

### Background Art

Amino acid is an essential component for human body and the growth of animals, and a new value relating to health functionality in various aspects has been found in recent years in addition to its nutritional value. Accordingly, food, drink and pharmaceutical products containing amino acid are awaited. For example, cystine ingested over a given amount has been found to be effective for the prevention of infection and the like, and expected to afford health food and health drink. In the case of slightly soluble amino acid having low solubility in water, however, a necessary amount thereof cannot be dissolved even when addition to a drink is desired, and uniform and a stable dispersion of slightly soluble amino acid is also difficult since it generally has high specific gravity (e.g., absolute specific gravity of cystine is 1.6) and high precipitation property. Accordingly, none of the conventional enteral feeding products and infusions containing poorly water-soluble amino acid contain a large amount of amino acid beyond its solubility.

While attempts have been made by employing methods such as addition of a thickener to increase viscosity of a medium, suppression of precipitation by addition of a fibrous support and the like to a medium, addition of a dispersing agent and the like to achieve uniform dispersion and the like, these methods give rise to problems such as low mixing property of liquid, low texture for ingestion as a drink, markedly degraded transparency of suspension, easy precipitation by a treatment such as heating and the like. Alternatively, the stability of quality may be degraded as evidenced by inconsistent content of a product in the production step and the like. Particularly, when added to a drink, precipitated amino acid causes difficult ingestion, or particularly, lower quality reliability. The references relating to the "Background Art" are as follows.
1. JP-A-2003-47871 .
2. JP-A-2003-47870
3. Powder performance of prototype continuous annular type wet medium mill (first report), Mitsuo KAMIWANO et al., SHIKIZAI KYOKAISHI, VOL. 66, 523-528 (1993)
4. Powder performance of prototype continuous annular type wet medium mill (second report) - influence of various grinding factors on grinding performance -, Mitsuo KAMIWANO et al., SHIKIZAI KYOKAISHI, VOL. 66, 529-533 (1993)
5. Indication and evaluation method of grinding progression state of fine particles in continuous annular type wet medium mill, Mitsuo KAMIWANO et al., Chemical Engineering Papers, 25, 796-802 (1999)

### Disclosure of the Invention

The problem to be solved by the present invention is provision of a suspension comprising a slightly soluble amino acid uniformly and stably dispersed therein, and a fine powder suitable for preparing the suspension.

The present inventors have conducted intensive studies in an attempt to solve the aforementioned problems and found that a fine powder having an average particle size of not more than a given level can be obtained by wet milling of a crystalline or amorphous slightly soluble amino acid such as cystine, aspartic acid, tyrosine, glutamic acid, tryptophan and the like, and the fine powder shows remarkably degraded precipitation property when suspended, whereby a uniform and stable suspension can be afforded, which resulted in the completion of the present invention.

Accordingly, the present invention provides the following.
(1) A fine powder of a slightly soluble amino acid having an average particle size of 0.1 - 5 µm.
(2) The fine powder of the above-mentioned (1), wherein the slightly soluble amino acid is one or more kinds selected from cystine, aspartic acid, tyrosine, glutamic acid and tryptophan.
(3) The fine powder of the above-mentioned (1), wherein the slightly soluble amino acid is cystine.
(4) A suspension of the fine powder of the above-mentioned (1).
(5) The suspension of the above-mentioned (4), wherein the slightly soluble amino acid is one or more kinds selected from cystine, aspartic acid, tyrosine, glutamic acid and tryptophan.
(6) The suspension of the above-mentioned (4), wherein the slightly soluble amino acid is cystine.
(7) The suspension of any one of the above-mentioned (4) - (6), wherein the concentration of the slightly soluble amino acid is 10 mg/100 g suspension - 30 g/100 g suspension.
(8) The suspension of any one of the above-mentioned (4) - (7), which comprises a dispersing agent.
(9) The suspension of the above-mentioned (8), wherein the dispersing agent is one or more kinds selected from lecithin, methylcellulose and pectin.
(10) The suspension of the above-mentioned (8) or (9), wherein the dispersing agent is added in an amount of 0.2% - 20% relative to the amount of the suspended amino acid.
(11) A method of producing the suspension of any one of the above-mentioned (4) - (10), which comprises wet milling a slightly soluble amino acid in the presence of water or an organic solvent.
(12) The production method of the above-mentioned (11), wherein the milling is performed in the presence of a dispersing agent.
(13) The production method of the above-mentioned (12), wherein the dispersing agent is added in an amount of 0.2% - 20% relative to the amount of the suspended amino acid.
(14) A method of producing the fine powder of any one of the above-mentioned (1) - (3), which comprises wet milling a slightly soluble amino acid in the presence of water or an organic solvent, and drying the amino acid by removing the water or organic solvent.
(15) A drink or food comprising the fine powder of any one of the above-mentioned (1) - (3) or the suspension of any one of the above-mentioned (4) - (10).
(16) The drink or food of the above-mentioned (15), wherein the slightly soluble amino acid is cystine.

### Brief Description of the Drawings

Fig. 1 is a graph showing changes in the cystine particle size distribution depending on the presence or absence of lecithin.
Fig. 2 is a graph showing changes in the cystine particle size distribution of the lecithin-10% system.
Fig. 3 is a graph showing changes (1) in the cystine particle size distribution of the methylcellulose-3% system.
Fig. 4 is a graph showing changes (2) in the cystine particle size distribution of the methylcellulose-3% system.
Fig. 5 is a graph showing changes in the cystine average particle size depending on the milling treatment time.

### Best Mode for Embodying the Invention

In the present invention, "slightly soluble" refers to an amino acid hardly soluble in water, organic solvents such as ethanol and the like, and a mixture thereof. In the present invention, "slightly soluble" means a solubility of not more than about 1 g/100 g solvent (20°C).

Examples of the "slightly soluble amino acid" in the present invention include cystine, aspartic acid, tyrosine, glutamic acid, tryptophan and the like, preferably cystine, aspartic acid and tyrosine, particularly preferably cystine.

In the present invention, "suspension" means a suspension wherein a fine powder of slightly soluble amino acid of the present invention is dispersed in a medium such as water, ethanol and the like, or a mixture thereof, preferably an aqueous suspension.

A preferable aqueous suspension in the present invention may contain, besides water to be the medium, salts, buffer, organic solvent and the like.

The slightly soluble amino acid before pulverization may be crystalline or amorphous, preferably crystalline.

The "average particle size" of fine powder in the present invention means, for example, a mass median particle diameter d₅₀, which is a minus sieve (%) of 50% as measured using a laser scattering type particle distribution measuring instrument (e.g., SK Microanalyzer PR07100 manufactured by Seishin Enterprises K.K.).

To uniformly and stably disperse a slightly soluble amino acid such as cystine, aspartic acid, tyrosine, glutamic acid, tryptophan and the like (average particle size about 20 - 50 µm as crystal of amino acid) in an aqueous medium, the present inventors considered forming a fine powder and tried dry-milling. However, the average particle size achieved by dry-milling is about 5 - 10 µm at most, and poorly water-soluble amino acid having a particle size of this level could not be uniformly and stably dispersed in water. When pulverized beyond this level, unpreferably, amino acid is highly likely denatured due to heat generation.

Thus, the present inventors tried wet milling and first found that slightly soluble amino acid such as cystine and the like can be obtained as a fine powder having an average particle size of about 0.1 - 5 µm, and that the fine powder is suitable for forming a stable suspension. The fine powder of the present invention is uniformly dispersed in a medium such as water, ethanol and the like, or a mixture thereof and the like to give a stable suspension.

In the present invention, as a wet milling machine used for wet milling for the production of a fine powder, any wet milling machine such as an apparatus for rotating a rotating-cylinder or a disk in a cylindrical container, preferably, a continuous annular type beads mill (e.g., spike mill (trade name) manufactured by INOUE MFG., INC. (SHG-4 vessel, volume 860 ml)), in which a cylinder with a protrusion is rotated, can be used.

The pulverized conditions are, for example, flow (200 ml/min), interior shaft rotation speed (2,900 rpm), beads packing rate 50%, circulation 9 times, temperature: start of pulverization 27°C, average during pulverization 38°C.

The concentration of amino acid in the medium during wet milling is determined from the aspects of industrial efficiency of pulverization and effectiveness during pulverization, and varies depending on the conditions of desired pulverization and the kind of amino acid to be pulverized. Amino acid is added to a medium in a proportion of 0.1 - 30 wt%, preferably about 2 - 20 wt%. When the concentration is higher than this range, the viscosity of the suspension increases and when it is lower, the efficiency of pulverization decreases. The amino acid concentration of a medium can be determined from the weight of amino acid added to the liquid. Alternatively, a sample of the suspension is prepared, which is dissolved by an appropriate method such as addition of acid or alkali, and the like, and the resulting solution is subjected to a general analysis method such as an amino acid analyzer and the like, whereby the amino acid concentration can be determined.

Including dilution after wet milling, a slightly soluble amino acid concentration of the suspension is, for example, 10 mg/100 g suspension - 30 g/100 g suspension, preferably, 10 mg/100 g suspension - 20 g/100 g suspension.

The viscosity can be measured, for example, using a rotary disk viscometer (DVM-B, manufactured by Tokyo Keiki Co., Ltd.), and an apparatus capable of measuring the viscosity of a non-Newtonian liquid by modifying the rotation conditions can also be used.

The measurement conditions include the use of, for example, rotor B (30 rpm, 50 sec, 20°C).

An amino acid crystal generally having an average particle size of about 20 - 50 µm can be processed into a fine powder having an average particle size of about 0.1 - 5 µm, preferably 0.1 - 3.5 µm, by wet milling. In the case of the amino acid, which is a soft organic product permitting easy increase of viscosity, unlike inorganic products, a further attempt to pulverize finely gives rise to a difficulty such as increased viscosity due to surface friction and the like.

In the present invention, a medium (preferably water) may be added for wet milling. Preferably, a dispersing agent is added. While the dispersing agent may be added during wet milling, it may be added to a suspension after pulverization rather than during pulverization. When a dispersing agent is added during pulverization, an increase in the viscosity can be suppressed during wet milling, thus enabling finer pulverization, and dispersion stability of a fine powder can be enhanced during preservation.

For example, in Example 2 mentioned later wherein a dispersing agent was not added, the viscosity during pulverization was 94.2 mPa·s (measured for a sample under pulverization), and pulverization was difficult to continue. In contrast, when the dispersing agent (lecithin) shown in Example 3 mentioned later was added, the viscosity during pulverization was 15.7 mPa·s, clearly showing promoted pulverization. The viscosity in these Examples was measured using a rotary disk viscometer (DVM-B, manufactured by Tokyo Keiki CO., Ltd.), and the measurement conditions included the use of rotor B (30 rpm, 50 sec, 20°C).

Examples of the dispersing agent to be used in the present invention include lecithin, methylcellulose, pectin and the like, and preferred are lecithin and methylcellulose.

The "dispersing agent" refers to a substance whose hydrophobic structure has affinity for a part of the surface of a substance to be suspended, and whose hydrophilic structure has affinity for a solvent for dispersion (e.g., water), thus improving the affinity between the substance to be suspended and the solvent, or a substance that suppresses association of the substances to be suspended, thus improving the uniformity and stability of the suspension. For example, bipolar substances such as lecithin and the like, water-soluble polysaccharides such as methylcellulose and the like, and the like can be mentioned. While the amount of the dispersing agent to be added varies depending on the kind thereof, properties of the solvent, and subject of addition, it is generally about 0.2 - 20%, preferably about 1 - 10%, relative to the substance to be suspended. Depending on the conditions, addition of a different amount may be appropriate.

Hard particles may be co-present during wet milling. Hard particles have higher hardness than the substance to be pulverized, or comparatively high specific gravity that enables application of a sufficient pulverization energy to substance particles to be substantially pulverized. For example, zirconium can be used. The particle size of hard particles is, for example, 100 - 700 µm, preferably 400 µm. While a suitable amount to be added varies depending on the conditions of hard particles and the target substance, it is, for example, about a half the volume of the pulverization container.

A suspension after wet milling can be directly or after appropriate dilution added to a drink, other food or a pharmaceutical product and the like.

The fine powder of slightly soluble amino acid of the present invention can be obtained by desolvation of a suspension obtained by wet milling. The drying method varies depending on the kind of the medium of a suspension and the like. Any method such as drying under reduced pressure, freeze-drying, air drying after casting on a plate and the like can be used, with preference given to freeze-drying.

The fine powder of the present invention can be added to a drink, other food or a pharmaceutical product and the like. In addition, various preparations can be produced by a general preparation technique comprising appropriate granulation processing to give granules and the like, or filling in a capsule and the like.

The amount of the fine powder and/or suspension of the present invention to be added to a drink, other food or a pharmaceutical product and the like is an amount necessary and sufficient for each amino acid to express its given function. Especially when added to a drink, a liquid food or a liquid pharmaceutical product, each amino acid is stably suspended in a fine particle state. Therefore, transparency and low viscosity can be maintained even at a high concentration, which is preferable from the aspect of preference.

The suspension and fine powder of the present invention contain an amino acid in a fine particle state, and can stably and remarkably improve bowel absorption in mammals inclusive of human. Therefore, they can effectively and stably maintain a particular function or treatment effect, and are useful for the maintenance of good health or therapy.

### Examples

The present invention is explained in detail in the following by referring to Examples, which are not to be construed as limitative.

The spike mill used in the Examples was SHG-4 manufactured by INOUE MFG., INC. (pulverization air volume 75 ml, vessel volume 860 ml) and the treatment conditions were 200 ml/min, interior shaft rotation speed 2,900 rpm, beads diameter 400 µm. The particle size distribution was measured using a laser scattering type particle distribution measuring instrument (manufactured by Seishin Enterprises K.K.: SK Microanalyzer PRO7100). For the dispersion stability of a suspension, the suspension and a 10-fold diluted solution thereof were stood still, and the precipitation state (precipitation rate, precipitate volume) of amino acid was observed.

### Example 1

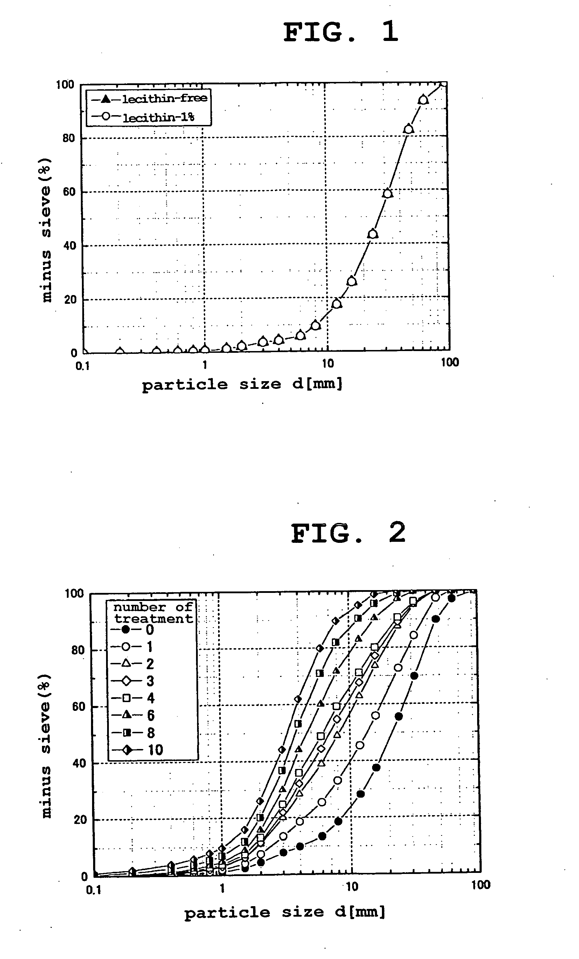

The difference in particle size distribution was examined based on the presence or absence of a dispersing agent. A spike mill was not used. A sample contained cystine (600 g), lecithin (0 g or 6 g) and water (5400 g). Fig. 1 shows the particle size distribution (average particle size 25 µm) when lecithin was not added and when 1 wt% of lecithin was added to cystine. They showed similar results where a mere addition of a dispersing agent did not change the particle size. Therefore, it was clarified that the both were not aggregates in which cystine could be crushed by the dispersing agent, but require division into fine particles by pulverization for stable suspending.

### Example 2

A sample containing cystine (600 g) relative to water (5400 g) was prepared and treated with a spike mill.

Although the viscosity increased as the number of treatments increased, the average particle size d₅₀ became smaller, which can be evaluated to show division into fine particles in progress. Particles having an average particle size d₅₀ of 3 µm could be obtained by 9 repeats of treatment. The suspension after pulverization was stable for about 1 week and did not show any precipitation.

### Example 3

A sample containing cystine (600 g) and lecithin (60 g) (10 wt% relative to cystine) and water (5400 g) was prepared and treated with a spike mill in the same manner as in Example 2.

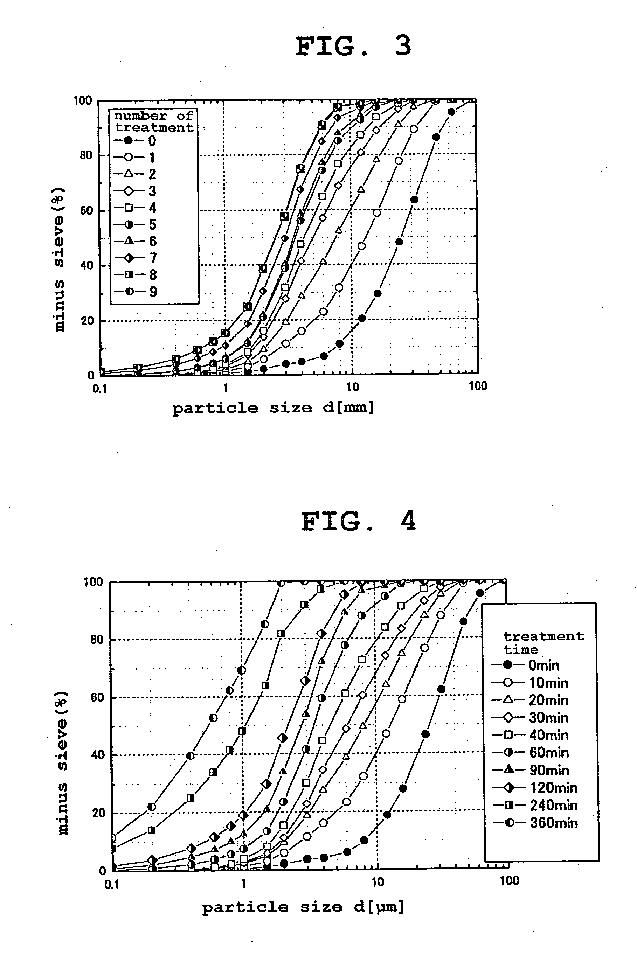

Fig. 2 shows changes in the particle size distribution when the sample was subjected to a dispersion test. The average particle size d₅₀ became smaller as the number of treatments increased, which can be evaluated to show division into fine particles in progress. Since an increase in the viscosity was small as the number of treatments increased, the addition of lecithin is considered to have promoted the pulverization.

The dispersion stability of a diluted solution obtained by adjusting the suspension (number of treatments 9) to 1% concentration showed improvement since the volume of precipitate decreased as compared to non-addition of lecithin. However, the time necessary for the precipitation was found to be short. This is considered to be attributable to the suppressed increase in the viscosity of the dispersion.

However, when this was added to a commercially available drink (Calpis Water (registered trademark)) containing a dispersing agent such as pectin and the like (final 0.3% relative to solution), precipitation was not observed after standing still for not less than 1 day. It was shown that a smaller particle size could afford practical dispersion stability.

### Example 4

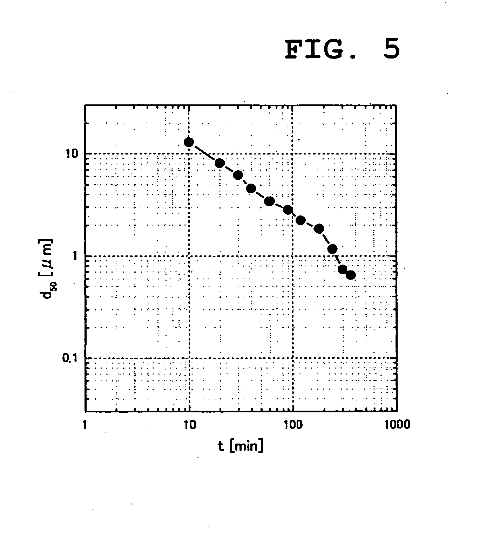

A sample containing cystine (600 g) and methylcellulose (18 g, 3% relative to cystine) and water (5400 g) was prepared and treated with a spike mill in the same manner as in Example 2. With regard to a dispersion experiment using methylcellulose as a dispersing agent, Fig. 3 shows changes in the particle size distribution for each number of treatments, Fig. 4 shows changes in the particle size distribution for each treatment time, and Fig. 5 shows an average particle size profile for the treatment time.

Particles having an average particle size d₅₀ of 2.4 µm could be obtained by 8 repeats of treatment. The viscosity hardly increased as the number of treatments increased. The suspension after pulverization was stable for about 1 week and did not show any precipitation.

As regards the dispersion stability of a diluted solution obtained by adjusting the suspension to 1% concentration, the amount of precipitation was lowered as compared to non-addition of a dispersing agent or use of lecithin instead of methylcellulose as a dispersing agent. The solution was mostly stable under the conditions of standing still for not less than 1 day. The stability was further improved by the addition of a dispersing agent such as pectin and the like.

### Industrial Applicability

According to the present invention, useful slightly soluble amino acids such as cystine, aspartic acid, tyrosine, glutamic acid, tryptophan and the like, which have been conventionally difficult for addition at a high concentration from the aspects of solubility and suspending property, can be uniformly and stably suspended in a drink, a liquid food or a pharmaceutical product and the like, and a suspension of the amino acid can be effectively used for a drink, a liquid food, a pharmaceutical product and the like. The suspension of the present invention can be used for the production of a drink, a liquid food, a pharmaceutical product and the like, which contain nutrition and a slightly soluble amino acid in an amount necessary and sufficient for the expression of nutrition and a particular function, at a high concentration and in a uniform dispersion state. Moreover, since the slightly soluble amino acid fine powder or suspension of the present invention is superior in bowel absorption in mammals inclusive of human, and can effectively expression a desired nutrition and a particular function, it is extremely useful in the fields of food and pharmaceutical products.

Using the suspension of the present invention, for example, amino acid superior in nutrition and particular health function can be contained in a drink or food, especially a drink having low viscosity and high transparency such as tea, sports drink and the like, or a food required to have transparency as well as texture such as jelly, gelidium jelly and the like, in a sufficient amount necessary from the aspects of nutrition and health. In addition, realization of uniform liquid dispersion state in a low viscosity liquid is also important for the quality management aiming to contain a given amount of amino acid indicated on each package unit in the production process of a food, drink or pharmaceutical product.

This application is based on a patent application No. 2004-354391 filed in Japan, the contents of which are incorporated in full herein by this reference.

## Claims

1. A fine powder of a slightly soluble amino acid having an average particle size of 0.1 - 5 µm.

2. The fine powder of claim 1, wherein the slightly soluble amino acid is one or more kinds selected from cystine, aspartic acid, tyrosine, glutamic acid and tryptophan.

3. The fine powder of claim 1, wherein the slightly soluble amino acid is cystine.

4. A suspension of the fine powder of claim 1.

5. The suspension of claim 4, wherein the slightly soluble amino acid is one or more kinds selected from cystine, aspartic acid, tyrosine, glutamic acid and tryptophan.

6. The suspension of claim 4, wherein the slightly soluble amino acid is cystine.

7. The suspension of any one of claims 4 to 6, wherein the concentration of the slightly soluble amino acid is 10 mg/100 g suspension - 30 g/100 g suspension.

8. The suspension of any one of claims 4 to 7, which comprises a dispersing agent.

9. The suspension of claim 8, wherein the dispersing agent is one or more kinds selected from lecithin, methylcellulose and pectin.

10. The suspension of claim 8 or 9, wherein the dispersing agent is added in an amount of 0.2% - 20% relative to the amount of the suspended amino acid.

11. A method of producing the suspension of any one of claims 4 to 10, which comprises wet milling a slightly soluble amino acid in the presence of water or an organic solvent.

12. The production method of claim 11, wherein the milling is performed in the presence of a dispersing agent.

13. The production method of claim 12, wherein the dispersing agent is added in an amount of 0.2% - 20% relative to the amount of the suspended amino acid.

14. A method of producing the fine powder of any one of claims 1 to 3, which comprises wet milling a slightly soluble amino acid in the presence of water or an organic solvent, and drying the amino acid by removing the water or organic solvent.

15. A drink or food comprising the fine powder of any one of claims 1 to 3 or the suspension of any one of claims 4 to 10.

16. The drink or food of claim 15, wherein the slightly soluble amino acid is cystine.
